# EUROPEAN PATENT SPECIFICATION

(11) **EP 2 783 001 B1**
(45) Date of publication and mention of the grant of the patent: **03.01.2018**
(21) Application number: 12852118.4
(22) Date of filing: 23.11.2012
(51) Int. Cl.: C12N 15/10, C12Q 1/68

(54) **MULTIPLEX ISOLATION OF PROTEIN-ASSOCIATED NUCLEIC ACIDS**
MULTIPLEXISOLIERUNG VON PROTEINASSOZIIERTEN NUKLEINSÄUREN
ISOLATION MULTIPLEX D'ACIDES NUCLÉIQUES ASSOCIÉS À DES PROTÉINES

(30) Priority: 22.11.2011 US 201161629555 P
(43) Date of publication of application: 01.10.2014
(73) Proprietor: Active Motif, Carlsbad, CA 92008 (US); Fernandez, Joseph, Carlsbad, CA 92008 (US); Jelinek, Mary, Anne, Carlsbad, CA 92008 (US); Egan, Brian, Stanley, Carlsbad, CA 92008 (US)
(72) Inventor: FERNANDEZ, Joseph, Carlsbad, CA 92008 (US); JELINEK, Mary, Anne, Carlsbad, CA 92008 (US); EGAN, Brian, Stanley, Carlsbad, CA 92008 (US)
(74) Representative: Schiweck, Weinzierl & Koch Patentanwälte Partnerschaft mbB
(86) International application number: PCT/US2012/066472
(87) International publication number: WO 2013/078470

(56) References cited:
- WO-A1-2010/048605
- WO-A2-2004/094456
- US-A1- 2002 051 986
- US-A1- 2005 130 161
- US-A1- 2010 240 101
- US-A1- 2013 011 833
- TERRENCE S. FUREY: "ChIP-seq and beyond: new and improved methodologies to detect and characterize protein-DNA interactions", NATURE REVIEWS GENETICS, vol. 13, no. 12, 1 December 2012 (2012-12-01), pages 840-852, XP055235590, GB ISSN: 1471-0056, DOI: 10.1038/nrg3306
- Anonymous: "ChIP-IT Express Magnetic Chromatin Immunoprecipitation Kits (version D2) Catalog Nos. 53008 & 53009", Active Motif Product Catalog 2009, 2009, pages FP-32, XP002755832, Retrieved from the Internet: URL:http://www.biotechniques.com/multimedi a/archive/00054/chip-it_express_manu_54059 a.pdf [retrieved on 2016-03-21]
- DOSTIE JOSÉE ET AL: "Mapping networks of physical interactions between genomic elements using 5C technology", NATURE PROTOCOLS, vol. 2, no. 4, 9 October 2009 (2009-10-09) , pages 988-1002, XP001525267, ISSN: 1750-2799, DOI: 10.1038/NPROT.2007.116 [retrieved on 2007-04-19]
- LIEBERMAN-AIDEN EREZ ET AL: "Comprehensive Mapping of Long-Range Interactions Reveals Folding Principles of the Human Genome", SCIENCE, vol. 326, no. 5950, 1 October 2009 (2009-10-01), pages 289-293, XP002591649, AMERICAN ASSOCIATION FOR THE ADVANCEMENT OF SCIENCE, US ISSN: 0036-8075
- KIDDER B.L. ET AL.: 'ChIP-Seq: technical considerations for obtaining high-quality data.' NATURE IMMUNOLOGY vol. 12, no. 10, 2011, pages 918 - 922, XP055240604
- DEMATTEI M.-V. ET AL.: 'Site-directed integration of transgenes: transposons revisited using DNA-binding-domain technologies.' GENETICA vol. 138, 2010, pages 531 - 540, XP019789321

## Description

### BACKGROUND OF THE INVENTION

### Overview of Epigenetic Mechanisms

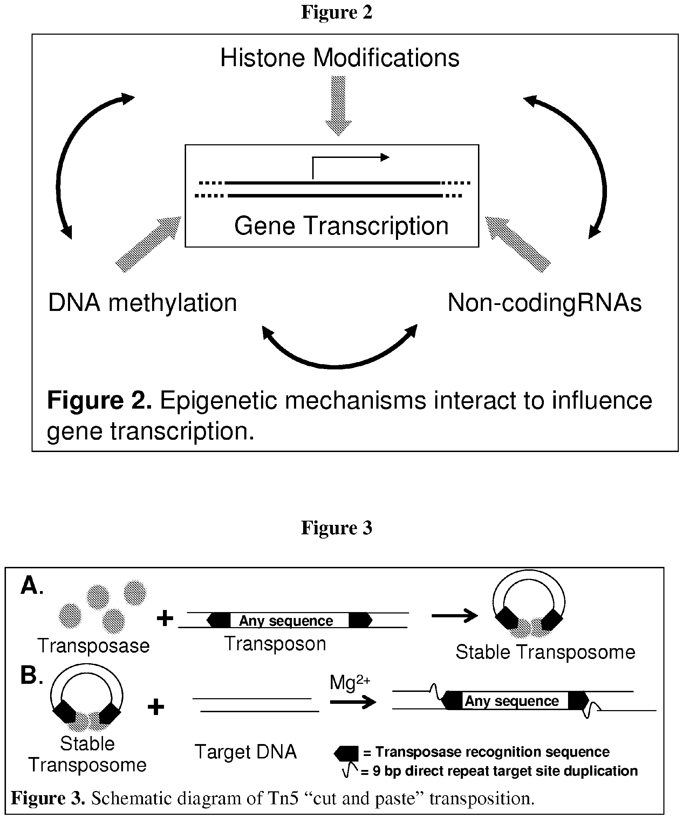

Epigenetics is broadly defined as changes in phenotype that are heritable but do not involve changes in the DNA sequence, and, from a historical perspective, stems from long-standing studies of seemingly anomalous (i.e., non-Mendelian) and disparate patterns of inheritance in many organisms [1]. Examples include variation of embryonic growth, mosaic skin coloring, random X inactivation, and plant paramutation. Discoveries in a large number of different model systems have been pivotal in identifying the three principle epigenetic mechanisms of (i) histone modifications, (ii) DNA methylation, and (iii) non-coding RNAs, which function in concert to influence cellular processes such as gene transcription, DNA repair, imprinting, aging, and chromatin structure, as depicted in Figure 2.

Gene transcription occurs in the context of the nucleosomal structure of chromatin. A nucleosome consists of an octamer of histone proteins (two molecules of each core histone H2A, H2B, H3, and H4) around which is wrapped 147 base pairs (bp) of DNA. Histones are small basic proteins with an unstructured amino-terminal "tail" that are the target of numerous post-translational modifications [2, 3]. Specific histone marks in the fission yeast *Saccheromyces pombe* were demonstrated to be directly operating as activating and repressing signals for gene transcription [4]. Methylation of lysine 4 and acetylation of lysine 9 of histone H3 are associated with transcriptionally active chromatin, while methylation of lysine 20 of histone H4 and methylation of lysine 9 and 27 of histone H3 are repressive marks, found in transcriptionally silent heterochromatin regions [5, 6]. The repressive histone H3 lysine 9 trimethyl-mark is bound by HP1 proteins, which in turn recruit non-coding RNAs involved in regulating heterochromatin formation [7].

Similar mechanistic links have also been identified between histone marks and DNA methylation. Highly repetitive DNA tandem repeat sequences such as those found in pericentric heterochromatin rely on the repressive H3K9 methylation mark to direct *de novo* DNA methylation while at promoters, EZH2, a histone lysine methyltransferase containing complex is involved [8]. Members of the methyl-CpG binding domain (MBD) family of proteins which are readers of DNA methylation are found in complexes with histone modifying enzymes (MeCP2 recruits histone deacetylases to mediate histone repressive marks [9]). Studies in multicellular organisms such as the invertebrates *Caenorhabditis elegans* and *Drosophila melanogaster* and plants such as *Arabidopsis thaliana* have generated crucial links between these epigenetic mechanisms [10].

In spite of all the advances to date, however, the epigenetics research field is still in the discovery phase, with many mechanistic questions remaining unanswered and many key players yet to be identified. Just as in the past, the continued study of epigenetic mechanisms in a variety of model organisms will be required to answer these questions. Development of enabling technologies suitable for a broad spectrum of model systems are also critical for accelerating the rate of discovery, especially since the various epigenetic mechanisms are functionally interconnected.

### Chromatin Immunoprecipitation (ChIP)

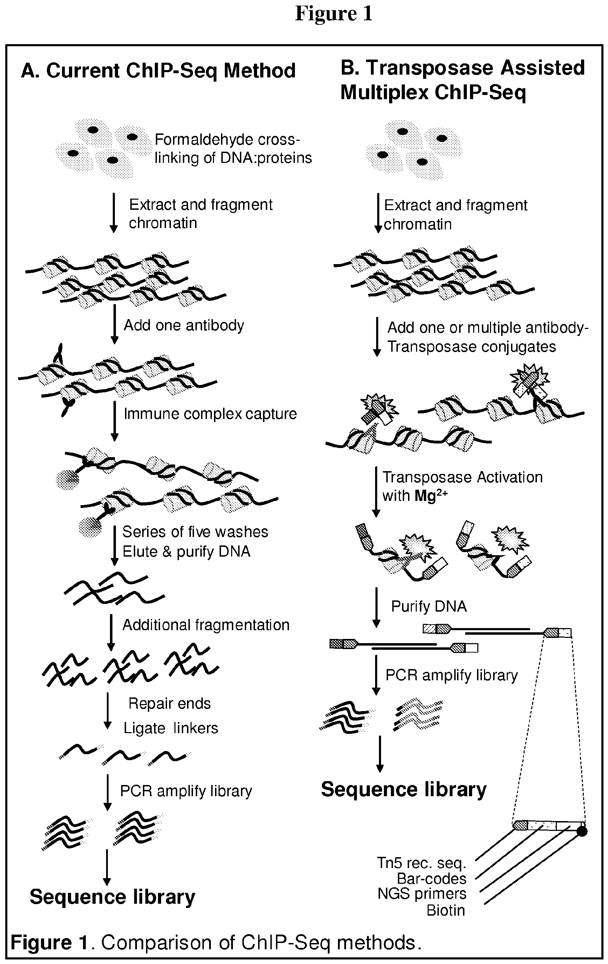

ChIP was first described in 1993 following studies of the association of histone acetylation state with transcriptional gene silencing in yeast [11]. Its adaptation to mammalian cells was reported five years later, in 1998 [12]. Since its initial description, the technique has remained essentially unchanged. As described below and depicted in Figure 1, Panel A, DNA sequence analysis is performed on the fraction of DNA isolated by immunoprecipitation with antibodies specific to the protein of interest. This technique is used in a wide variety of applications. These include profiling histone modification patterns, from their intragenic distribution through to genome-wide analysis, determining the composition of protein complexes recruited by specific histone marks, identifying regions of *de novo* DNA methylation, or, with some modifications to the procedure, detecting nascent non-coding RNAs.

Advances in PCR and DNA sequencing technologies have positively impacted the DNA analysis portion of the ChIP technique, which has expanded from semi-quantitative analysis of single genes using end-point PCR, to quantitative analysis with real-time PCR, through to genome-wide analysis afforded by ChIP-chip, wherein the captured DNA is used to probe a high-density microarray, or ChIP-Seq, wherein the captured DNA is subjected to NGS ("next generation sequencing") [6, 13]. While these improvements have increased the magnitude of sequence information available for analysis from a single reaction, the limitations associated with efficient immunocapture of protein-associated DNA have not been addressed.

Only incremental improvements, such as the introduction of magnetic beads for immunocapture in place of agarose or sepharose beads, as in Active Motif's CHIP-IT Express™ kit, have been made [14]. The improved recovery (fewer beads are lost during wash steps), reduced background (wash steps are more thorough) afforded through the use of magnetic beads has allowed for a ten-fold reduction in the sample size requirements, from 2-10 million cells to 0.1-1 million cells. In general, these lower sample requirements apply only to high affinity antibodies targeting abundant proteins, such as RNA polymerase II or histone modifications. In addition, the sample size requirement remains a considerable barrier in some research areas, such as embryology and stem cells where cell numbers are very limiting, and is further compounded by the limitation that the only a single protein can be analyzed in each ChIP experiment. The number of cells required is thus directly proportional to the number of proteins to be analyzed, impacting cost and time considerations. An additional challenge stems from the need of ultra-high affinity antibodies for use in this technique. Many antibodies qualified for use in immunofluorescence and/or immunohistochemistry, which can be used to demonstrate *in situ* association of the protein of interest with DNA or chromatin, or antibodies which have been shown to effectively function in immunoprecipitation, fail in ChIP applications where the target protein is present in high molecular weight multi-protein-chromatin complexes containing DNA fragments up to 1 kb (kilobase) in length. The binding affinity of the antibody for its cognate target must be strong enough to withstand the physical forces associated with constant agitation of the suspension and immobilization by the beads used to isolate the complexes.

### Need for and Benefits of the Invention

The instant invention has broad and significant practical applications. These applications span all life sciences research with eukaryotic organisms, because epigenetic mechanisms are highly conserved throughout eukaryotes. The methods of this invention are more efficient than existing methods such as ChIP. These new, patentable methods enable concurrent analysis of multiple chromatin-associated proteins, eliminate the labor intensive NGS library preparation procedures, and have the potential to significantly reduce the amount of samples needed compared to traditional ChIP methods. This is relevant to not only to the stem cell and embryology research fields where samples are limiting, but also fields such as high throughput screening of large numbers of samples in clinical and pharmaceutical applications, where miniaturization is a major cost driver. In addition, ChIP analysis is limited by the small percentage of antibodies that work effectively in the method. Since the methods of the invention do not require immunoprecipitation, antibodies that do not work in ChIP can be adapted to work with the instant invention, thereby expanding the number of cellular proteins whose genomic distribution can now be determined.

### SUMMARY OF THE INVENTION

One aspect of the invention concerns methods and reagents for making a nucleic acid sequence library or libraries. Such methods involve extracting and fragmenting chromatin from a prepared sample, adding at least one antibody-oligonucleotide conjugate comprising an extraction moiety, allowing said antibody(ies) to locate at its/their target protein(s) in said chromatin fragments, tagging the nucleic acid in said chromatin fragments with said conjugate by inducing an intermolecular reaction between said oligonucleotide and said nucleic acid, extracting the nucleic acid so tagged using the extraction moiety.

In some embodiments, the antibody-oligonucleotide conjugate further comprises transposase and the intermolecular reaction is transposition, the extraction moiety is a biotin molecule, and/or the intermolecular reaction is selected from the group: transposition, ligation, recombination, hybridization, and topoisomerase-assisted insertion.

A related aspect of the invention concerns antibody-transposome complexes. Such complexes comprise an antibody that binds a target nucleic acid-associated protein conjugated to a transposome that comprises a transposase and a transposon cassette.

Another aspect of the invention relates to methods for performing proximity ligation. Such methods include contacting a cross-linked and fragmented chromatin sample with an antibody-oligonucleotide conjugate under dilute conditions to promote ligation of the ends of the chromatin fragment to the ends of the oligonucleotide of the antibody-oligonucleotide conjugate, wherein the oligonucleotide is double stranded and comprises at least two recognition sites for a freeing restriction enzyme, primer sites for amplification, at least one bar code sequence to identify the conjugated antibody, complementary overhangs to facilitate ligation, and optionally, a spacer for optimizing the length of the oligonucleotide, and then ligating the antibody-oligonucleotide conjugates to the cross-linked and fragmented chromatin sample.

A related aspect involves antibody-oligonucleotide conjugates useful for proximity ligation reactions. These typically comprises an antibody that binds a target nucleic acid-associated protein conjugated to a double-stranded oligonucleotide that comprises at least two recognition sites for a freeing restriction enzyme, primer sites for amplification, at least one bar code sequence to identify the conjugated antibody, complementary overhangs to facilitate ligation, and optionally, a spacer for optimizing the length of the oligonucleotide.

### BRIEF DESCRIPTION OF THE FIGURES

Figures 1-9 depict various aspects and embodiments of the invention.

### DETAILED DESCRIPTION OF THE INVENTION

This invention provides methods of tagging and isolating DNA or other nucleic acids that are associated with a protein or proteins of interest. Generally the methods comprise first preparing complexes of oligonucleotide tag(s) or barcode(s) with antibody(ies) that recognize protein(s) of interest in chromatin or that are otherwise associated with nucleic acids. The tagged oligonucleotide complexes may further comprise an extraction moiety, such as a biotin molecule (or other member of a high affinity binding pair), that can be used to extract or isolate the tagged nucleic acid. A "binding partner" or "member" of a high affinity binding pair (i.e., a pair of molecules wherein one of the molecules binds to the second molecule with high affinity (e.g., biotin and avidin (or streptavidin), carbohydrates and lectins, effector and receptor molecules, cofactors and enzymes, enzyme inhibitors and enzymes, and the like).

Next, when the complexes are added to the nucleic acids, the antibody(ies) recognize or bind to the protein(s) of interest that are associated with the nucleic acids. Using a variety of intermolecular reactions, the nucleic acid proximate those proteins is tagged with the complex. Thus, the proximate nucleic acid is tagged with one or more oligonucleotide bar code(s) and, optionally, a moiety that allows for purification or isolation.

One embodiment of the invention, termed "Transposase-Assisted Multi-analyte Chromatin ImmunoPrecipitation" or "TAM-ChIP", is a novel, patentable method that significantly improves CHIP, the principle technique currently used to study how histone post-translational modifications and the proteins which they recruit regulate gene expression. Traditional ChIP is a cumbersome multiday, multistep procedure that requires large numbers of cells, ultra-high affinity antibodies for the immunocapture of large protein-chromatin complexes, and is limited to the analysis of a single protein species per sample.

Briefly, conventional ChIP methods involve the cross-linking of DNA and protein in live cells, isolation of cross-linked material, shearing of DNA (still bound, through cross-linking, to protein), immunoprecipitation of the cross-inked DNA-protein complexes via antibody-binding of the protein of interest (still bound to DNA), reverse-cross-linking of DNA and proteins, and the detection or sequencing of DNA molecules that were cross-linked to the immunoprecipitated DNA-protein complexes, allowing the generation of specific, DNA sequence context data **(****Figure 1****, Panel A**). For ChIP-Seq applications, the elapsed time from formaldehyde cross-linking of cells to sequencing-ready library is typically five days. Relative to the advances made in the understanding the epigenetic mechanisms of DNA methylation and micro- or non-coding RNAs, the limitations of the ChIP technique have significantly hampered the understanding of the biological function of histone modifications.

In contrast, TAM- CHIP **(****Figure 1****, Panel B)** removes a number of technical and sample-size barriers associated with traditional ChIP by eliminating the inefficient immunoprecipitation and labor intensive library preparation steps of the method and bringing high throughput sample processing and multi-analyte capabilities to the ChIP method. TAM-ChIP enables rapid (<24 hour elapsed time) and streamlined analysis of one or several protein-chromatin interactions for analysis of either a single gene all the way through to genome-wide interrogation. To achieve this, antibodies specific for the protein(s) of interest are first conjugated to a transposase:transposon complex (Transposome™) charged with synthetic oligonucleotide(s) that comprise a transposon cassette containing the following features:
- Transposase recognition sequences required by the for catalysis of the DNA integration reaction;
- a biotin (or other) molecule conjugated to an oligonucleotide, preferably at one end, to facilitate purification of targeted DNA with streptavidin magnetic beads (or other suitable support conjugated to the other member of the selected high affinity binding pair);
- unique bar code sequences (i.e., short nucleotide sequences, i.e., from 1-1,000 bases, preferably 1-50 bases, preferably fewer than 20, even more preferably fewer than 10 bases) that uniquely label an oligonucleotide species so that it can be distinguished from other oligonucleotide species in the reaction, and which correspond to a particular antibody) for antibody identification in multi-analyte applications in which multiple antibodies are used simultaneously with the same sample material;
- for whole genome sequencing applications, platform-specific tags required for next generation sequencing (NGS).

The antibody-transposase conjugates are incubated with chromatin fragments extracted from isolated cells, tissue, or whole organs (or other cell-containing biological samples) to allow specific antibody-protein binding. The transposase is subsequently activated by addition of a cofactor, e.g., Mg²⁺, after sample dilution to prevent intermolecular events. Transposase activation results in random insertion of the two transposase-associated oligonucleotides into the antibody-associated DNA fragment, thereby producing analysis-ready templates following a deproteination step and capture of biotin-tagged DNA fragments using streptavidin-coated magnetic beads.

### Leveraging Tn5 Transposase for Improving ChIP

Transposable elements are discrete DNA segments that can repeatedly insert into a few or many sites in a host genome. Transposition occurs without need for extensive DNA sequence homology or host gene functions required in classical homologous recombination[15]. Consequently, transposable elements have proven to be superb tools for molecular genetics and have been used extensively *in vivo* to link sequence information to gene function. More recently, *in vitro* applications have also been developed, specifically for Tn5, a class II "cut and paste" transposable element isolated from gram negative bacteria [16]. Catalysis involves nicking of DNA to generate nucleophilic 3' OH groups on both strands at the ends of the 19 bp Tn5 transposase DNA recognition sequence. The 5' ends are also cleaved within the synaptic complex, releasing the transposable element from the donor DNA **(****Figure 3****, Panel A).** This mechanism allows for the formation of a stable complex between the enzyme and transposon in the absence of Mg²⁺ [17], and is the basis for the *in vitro* transposase technologies developed by Epicentre Biotechnology (Madison, WI, USA).

Transposases are not conventional enzymes in the classical sense, in that there is no turn-over. Spontaneous product release is not required and consequently the transposase is required in stoicheometric quantities [15].

Tn5-mediated transposition is random, causing a small 9 bp duplication of the target sequence immediately adjacent to the insertion site **(****Figure 3****, Panel B).** The result is analogous to using a restriction endonuclease with random sequence specificity that also contains a ligase activity. Epicenter's EZ-Tn5 Transposome™ technology utilizes a transposase-transposon complex which exhibits 1,000 fold greater activity than wild type Tn5, achieved by combining a mutated recombinant Tn5 transposase enzyme with two synthetic oligonucleotides containing optimized 19 bp transposase recognition sequence [16, 18], and is the basis of Epicentre's Nextera™ product used to streamline NGS library preparation. Using such a recombinant enzyme (whether naturally occurring or engineered to have improved transposition activity), transposition occurs with at efficiencies of 0.5-5%, using as little as 50 ng of purified DNA, yielding >10⁶ transpositions per reaction. The transposome is so stable that it can be introduced via electroporation into living organisms, both prokaryotic (Gram negative and Gram positive bacteria [19-22]) and eukaryotic (yeast, trypanosome, and mice [19, 23, 24]) where in the presence of endogenous Mg²⁺, transposon insertion has shown to be random and stable. The ability of the Tn5 transposase to recognize eukaryotic chromatin as a substrate is extremely significant, as it can be adapted to transform ChIP into a multi-analyte method suitable for high through-put applications.

As described above and depicted in **Figure 1****, Panel B,** TAM-ChIP technology development uses an antibody-transposome linking moiety to effectively conjugate the Transposome™ to a targeting antibody that binds a targeted DNA-associated protein. Binding of the antibody to its target protein in chromatin (or other nucleic acids with which the protein associates in cells under physiological conditions) optimizes transposase activity with chromatin as a DNA substrate. The TAM-ChIP method involves allowing formation of complexes between the antibody-Transposome™ conjugate and chromatin fragments containing the antibody's target protein. The samples are then diluted (to ensure transposition of the olignucleotide payload in the transposon cassette into the same DNA fragment) and the chromatin-associated-transposase activated by the addition of the Mg²⁺ co-factor, resulting in the insertion of the transposon cassette containing bar-code sequences and NGS compatible primer sites into flanking DNA regions **(****Figure 1****, Panel B).** Following DNA purification to remove proteins, PCR amplification (or another suitable amplification process) with primers complementary to the oligonucleotides in the can be performed to generate NGS compatible libraries for sequencing.

The direct insertion of the oligonucleotide duplex in the transposon cassette by the transposase eliminates the need for immunoprecipitation, thereby reducing the input DNA requirement. It can also eliminate the need for ultra-high affinity antibodies, thereby expanding the application of the ChIP technique to a broader range of cellular targets which were previously excluded due to the lack of suitable antibodies. The inclusion of barcode sequences in the oligonucleotides allows for the identification of the corresponding immunoprecipitating antibody, and is the basis of the multi-analyte potential of TAM-ChIP, which for the first time enables simultaneous use of multiple antibodies in the same sample and experiment. This innovation also has the benefits of further reducing sample size requirements and enables elucidation of protein co-association in sequence-specific contexts throughout the genome.

### Methods and Representative Examples

Preferred methods, materials, and conditions for carrying out some preferred, representative embodiments of the invention are described below. Those of ordinary skill in the art will readily appreciate that the invention can be practiced in a number of additional embodiments using equivalent alternate techniques and materials.

### Example 1: TAM-ChIP

### Preliminary Data

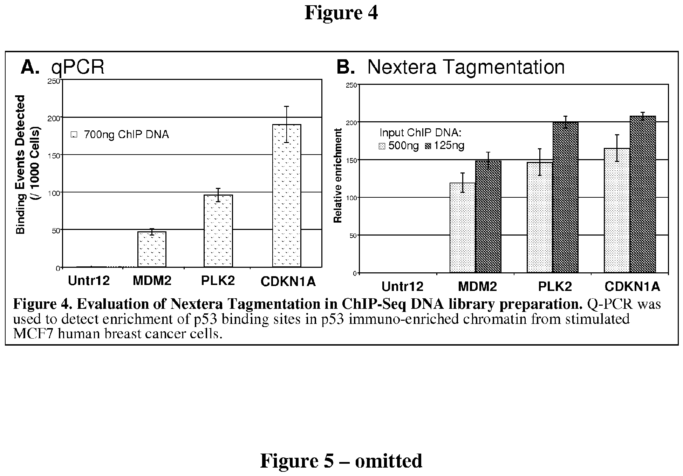

In order to improve the turnaround-time of conventional ChIP-Seq services, Epicentre's Nextera™ DNA Sample Prep kit, which uses the EZ-Tn5 Transposome™ and suppression PCR to generate NGS compatible libraries, was evaluated for suitability for use with ChIP-enriched DNA. ChIP was performed in duplicate using p53 antibodies and 30µg chromatin extracted from estrogen stimulated MCF-7 cells (a human breast cancer cell line) following established protocols, and isolated DNA was then purified. Quantitative PCR was performed on known p53 binding sites to validate the specificity of the anti-p53 ChIP reactions **(****Figure 4****, Panel A).** Untr12 is a negative control in a gene desert on human chromosome 12 and is not expected to be bound by p53.

The Nextera transposition reaction was performed using two quantities of CHIP DNA **(****Figure 4****, Panel B)** according to the manufacturer's protocol. The DNA libraries were purified and used for PCR according to the Nextera protocol for 18 cycles. The amplified DNA was purified and quantified by measuring absorbance at 260 nm (A260) using a NanoDrop spectrophotometer. The amount of DNA produced in the Nextera reaction was in the range of what is typically obtained using the Illumina library protocol.

These data demonstrate the suitability of EZ-Tn5 for use with fragmented DNA substrates, and that the p53 binding sites detected in traditional ChIP are preserved and quantifiable in Nextera-generated libraries. Interestingly, a higher amount of DNA was generated in the Nextera reaction with the smaller amount of DNA isolated by ChIP, suggesting that the transposition efficiency was higher and that less input chromatin may be required for ChIP experiments when EZ-Tn5 is incorporated into the methodology.

For the methods described below, the EZ-Tn5 transposome is purchased from Epicentre Biotechnology (Madison, WI, USA) and ChIP-IT Express™ reagents and protocols are used (Active Motif, Carlsbad, CA, USA) as the ChIP reagents throughout this example. The end result is an optimized method for the ChIP-validated antibody-transposome conjugates.

The methods below are performed in human HeLa cell lines, which are easily cultured *in vitro* to produce the necessary quantities of genomic DNA (gDNA) or chromatin required for the experiments described below. While many epigenetic research tools and consumables target researchers using vertibrate animal model systems, largely because this segment is the

**Table 1. Candidate HeLA genomic loci for qPCR analysis of transposition efficiency**

| **Transcriptionally Active** | | | | |
|---|---|---|---|---|
| GAPDH | HOXA10 | EEF1A1 | TUB1C | LDHA |
| RASSF1A | ACTB | PPIB | PABPC1 | RPS18 |

| **Transcriptionally Repressed** | | | | |
|---|---|---|---|---|
| PTGER3 | HOXD13 | HBB | Untr12 | NGB |
| CFDP1 | Sat2A | MyoD | PAX2 | MYT1 |

largest in the epigenetic research tools market, the principle epigenetic mechanisms are conserved throughout vertebrates (including the primary amino acid sequence of histones and the repertoire of post-translational modifications), although thoise skilled in the art will be able to adapt the reagents and methods of this invention for use with other organisms. Another compelling reason for the use of mammalian cells for the TAM-ChIP technology stems from the complexity of the genome. ChIP is far more challenging in mammalian cells, where genes represent only 1-1.5% of the genome, than in lower eukaryotes where genes represent a much large fraction of the total genome (compare with 70% in S. *Cerevisiae*).

### Analytic Methods

The majority of the experiments described below require determination of transposition efficiency, and evalution of the distribution (both abundance and range) of DNA fragments generated as a consequence of transposition. Transposition efficiency can be determined using any suitable technique, for example, by quantitative real-time PCR using a StepOnePlus RT-PCR thermocycler (Applied Biosystems) and primers complimentary to a panel of genomic loci known to be either transcriptionally active or repressed in HeLa cells (Table 1, above) [25]. Transposition results in the insertion the biotin-tagged transposon oligonucleotide into the target DNA, enabling isolation of transposon-tagged DNA fragments with streptavidin-coated magnetic beads and subsequent quantitation in triplicate by real time PCR. A five-fold dilution series of fragmented HeLa genomic DNA can be used as standards to generate a quantitation curve. Identical locus-specific PCR primer sets are used for both samples and standards, and transposition efficiency will be calculated as the median of the DNA recovered for all loci. The generation of tagged fragments less than about 200 bp is particularly preferred to achieve the necessary resolution of sequence reads in NGS applications. Evaluation of the abundance and range of transposon tagged-DNA fragment sizes produced by transposition events requires, for example, an Agilent 2100 Bioanalyzer, which employs a microfluidics system for electrophoretic determination of size and quantity of DNA fragments in sample volumes of 1-4 µl.

### Transposase Tn5 with Chromatin Substrates

The majority of applications developed to date for the *in vitro* generated transposase-transposon complex use purified DNA as substrate, and the ability of the Tn5 transposase to utilize chromatin as substrate *in vivo* has been demonstrated. This example identifies the optimal chromatin extraction and fragmentation method and optimal reaction conditions to achieve maximal transposition efficiency. Transposition efficiency is be determined using quantitative real-time PCR to determine the number of integration events using transposon-specific primers.

Cross-linking of associated proteins to DNA with cell permeable chemicals such as formaldehyde is typically the first step performed in ChIP to assure preservation of DNA:protein interactions while the protein of interest is immunoprecipitated. Typically, cells are incubated for 10 minutes in the presence of 1-4% formaldehyde, formaldehyde is quenched by the addition of glycine, and cells lysed. Native ChIP is an alternate method that does not involve chemical cross-linking agents. Whole cell or nuclear lysates are then sonicated to achieve both improved solubilization and fragmentation of chromatin. Nuclear isolation reportedly reduces non-specific binding during the immunoprecipitation phase of the protocol, thereby improving readout signal to noise ratios. The approach described here is used to determine the effects of protein-DNA cross-linking with formaldehyde and chromatin fragmentation on transposase efficiency. Experiments are repeated on three independent occasions, with quantitative real-time PCR performed in triplicate for each condition.

### ChIP Buffer Dilution Evaluation

The composition of the buffers used to extract chromatin from cells contain harsh detergents such as sodium dodecylsulfate (SDS) and EDTA to inhibit nuclease activity. First, the extent to which ChIP buffers need to be diluted so as to preserve transposase activity are determined. Epicentre provides two proprietary reaction buffers for the transposition reaction. The low-molecular weight (LMW) and high-molecular weight (HMW) transposase buffers are used to produce fragment libraries of 200-1,000 bp and 200-2,000 bp respectively. Only the LMW buffer is used herein to achieve the DNA sequence read resolution required for ChIP-Seq. A mock ChIP experiment is performed following the ChIP-IT Express protocol to produce the buffer composition present in the chromatin immunocapture step, the step at which the transposome would be activated by the addition of Mg²⁺ in the TAM-ChIP method. A two-fold dilution series (ranging from 1:2 to 1:32) of this buffer is prepared in Epicentre's LMW buffer and transposase reactions are performed following manufacturer's established protocols with 50ng DNA. Reactions in which LMW buffer is spiked with increasing amounts of EDTA (5, 10 and 25 mM) serve as negative controls for transposase activity. Unmethylated lambda phage DNA (48.5 kb), and purified fragmented HeLa cell gDNA (>10 kb fragment size), are used as substrates. Integration efficiency and fragment size profiles of tagged-DNA are determined as described above. Transposition efficiency in the various buffers with the various substrates are reported as a percentage relative to the transposase efficiency observed with neat LMW assay buffer and lambda phage DNA. These data are used to identify the minimum dilution factor by which antibody-chromatin complexes should be diluted such that transposase activity (both transposition efficiency and DNA fragment profile) is unaffected by residual detergents and EDTA. The inclusion of purified Hela DNA enables establishment of a baseline reference for transposase activity with mammalian methylated DNA.

### Transposition with Chromatin as Target DNA

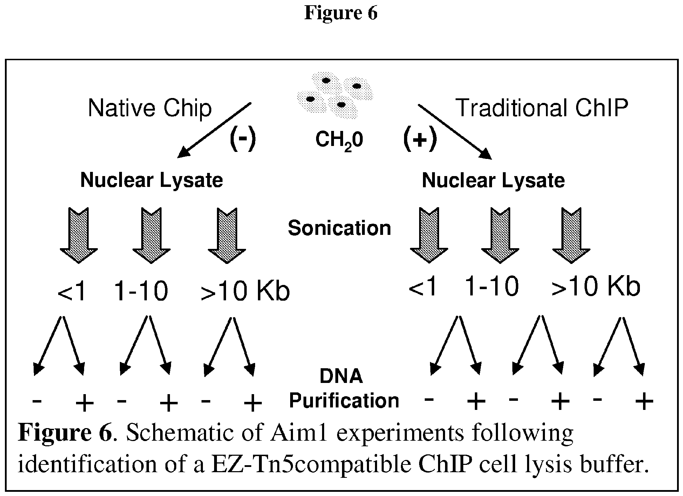

Figure 6 summarizes the experimental scheme for the identification of optimal transposition with chromatin substrates. Chromatin is extracted from untreated and cross-linked cells (1% formaldehyde for ten minutes) and DNA quantitated by measuring A260. Mechanical fragmentation of chromatin, for example, by sonication, is performed such that one third of the sample contains fragments <1000 bp (as in traditional ChIP), the second third contains fragments ranging between 1,000 and 10,000 bp, while the third sample contains fragments >10,000 bp. Unfragmented genomic DNA is too viscous for effective *in vitro* manipulations and enzymatic fragmentation does not resolve higher-order chromatin structures, whose presence would undermine interpretation of NGS results, and consequently, neither is preferably used herein.

DNA in one half of each of these samples is purified to enable comparison of transposition efficiency between chromatin and naked DNA. Chromatin samples (unpurified DNA) are diluted in LMW buffer using the minimum dilution factor determined above. 50ng chromatin (quantitated by A260) and 50ng purified DNA is used in transposition reactions using any suitable protocol. Transposition with purified lambda phage DNA is used as reference. Transposition efficiency and fragment size is determined as described above. If transposase activity is too low, the experiment is repeated with 200ng chromatin, or more as required. This method identifies which chromatin preparation results in the production of a population of fragments wherein greater than 40% are less than 200 bp in length. This preparation method is used subsequently in embodiments of the TAM-ChIP technology described below.

### Example 2: Antibody-Transposase Conjugates

TAM-ChIP requires that the enzymatic activity of the transposase preferably be unaltered, with regards to catalytic rate and randomness of integration sites, when coupled to another protein. Conjugations with various chemistries and cross-linkers of varying length are compared using ChIP validated antibodies. This example generates functional antibody-transposome conjugates.

An extensive number of ChIP-validated antibodies are commercially available or can be developed using conventional antibody production techniques. Here, antibodies to a chromatin associated protein (RNA polymerase II) and a structural chromatin protein, a histone (anti-histone H3 trimethyl-lysine 4 (H3K4tm) mark associated with transcriptionally active chromatin), are conjugated to the EZ-Tn5 transposome using any suitable approach, two of which are described below.

Antibodies can be chemically cross-linked either to the transposase (protein-protein) or to the transposon (protein-DNA) using HydraLink Chemistry (Solulink, San Diego, CA, USA), which is stoichiometrically more efficient than traditional EDC/NHS chemistries and has been used in the development of PCR-based proximity ligation assays, recognized as the most sensitive assay for protein detection [26-28]. The chemistry involves formation of reaction between an aromatic hydrazine (hydrazinonicotinamide-HyNic) and an aromatic aldehyde (4-formylbenzamide-4FB), yielding a stable bis-arylhydrazone that is UV-traceable, absorbing at 350nm. Conjugation reaction kinetics can be augmented 10-100 fold in the presence of aniline, leading to conjugation yields of >95%[26].

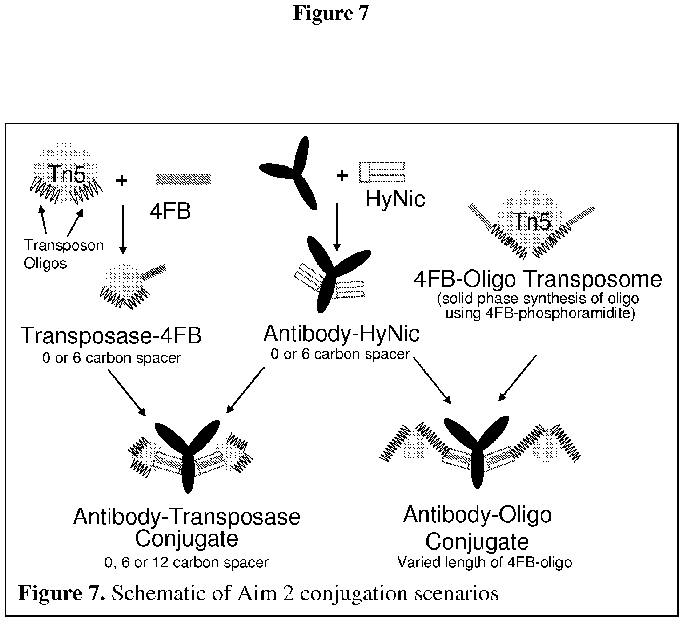

Conjugations are performed following the manufacturer's established protocols in quantities sufficient for their functional characterization described below and for their subsequent use in the methods described. Both antibody-transposase and antibody-transposon, the transposase-assoicated oligonucleotide (Figure 7) conjugates is prepared using varying cross-linker lengths (0, 6 or 12 carbon side-chains) for protein-protein conjugates or transpson oligonucleotides of varying lengths (synthesized with additional 20, 40 or 60bp), with the 4FB moiety incorporated during solid phase sythesis. Conjugate stoichiometry is determined by measuring absorption of the bis-arylhydrazone crosslinking product at 350 nm which has a molar extinction coefficient of 1600 M⁻¹ [29]. Aliquots reserved at each step are used to monitor transposase activity by measuring transposition effieciency with lambda DNA (as above) and retained antibody recognizition of antigen by dot blot analysis using established Active Motif protocols. This Example provides isolation of antibody-transposase conjugates with a stoichiometry of greater than or equal to two transposase molecules per antibody molecule in which the function of antibody and transposase is no less than 90% of their unconjugated counterparts. These conjugates are used below for the the TAM-ChIP technology. Methods for conjugation of antibodies to a variety of molecules (enzymes, dyes, oligonucleotides, biotin) are well established and are considered routine. Tn5 transpsosase fusion proteins have been described and are functional [30, 31]. Accordingly, any suitable approach can be adapted for use in the context of this invention.

### Example 3: TAM-ChIP Optimization

Examples 1 and 2 above provides the basis for performing TAM-ChIP and demonstrating its benefits relative to traditional ChIP methods. The optimized chromatin extraction and fragmentation procedure above is combined with the antibody-transposome conjugate to perform the TAM-ChIP procedure. A method of comparing the genomic representation of the sequencing libraries produced by TAM-ChIP and traditional ChIP-Seq is also provided. This is done using two steps. The first step involves optimizing sets of conditions with regards to chromatin and antibody-transposase concentrations, optimization of incubation times using transposition the analytic methods describe above as the readout. The second step is a direct comparison of the genomic representation of the DNA libraries produced by TAM-ChIP with that of conventional ChIP-Seq methods.

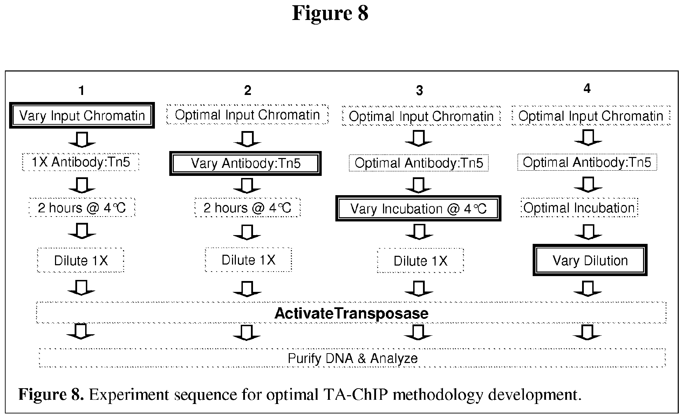

An optimal protocol can be determined using the steps depicted in **Figure 8****.** First, the optimal amount of chromatin substrate is determined, as this impacts both transposition efficiency and fragment size. Initially, antibody-transposase conjugates are used at a fixed amount, where in the amount of transposase enzyme present corresponds to the amount recommended in applications developed by Epicentre Biotechnology for use with 50ng DNA.

Triplicate samples of 50, 150, and 450ng of HeLa cell chromatin (quantitated by A260) are incubated with the antibody-transposase conjugate in 100 µl for two hours at 4°C **(****Figure 8****, column 1**). The chromatin-antibody complexes are diluted in LMW buffer and transposase activated by the addition of 10mM Magnesium acetate using the optimized transposase reaction conditions developed above. Samples are be treated with 20µg Proteinase K for 1 hour at 37°C and DNA purified using a Zymo DNA Clean & Concentrator-5 Kit (or equivalent). If formaldehyde cross-linked chromatin is used, prior to protease treatment, reversal of cross links is achieved by the addition of an equal volume of Reverse Cross-Linking Buffer (50mM NaCl in 50mM Glycine) and samples incubated at 95°C for 15 min.

Biotin-tagged DNA fragments are captured using streptavidin magnetic beads and transposition efficiency and fragment size profiles are determined as described above. Transposition efficiency is significantly higher at the transcriptionally active genomic targets listed in **Table 1** than at the transcriptionally silent regions that are analyzed by qPCR. Consequently, for these experiments transposition efficiency is calculated as a relative ratio of transposition into transcriptionally active and inactive regions, thereby providing a means for comparison of the specificity and efficacy of the antibody-transposome complexes. The range of input chromatin is expanded in subsequent experiments if transposition efficiencies are too low or tagged-DNA fragments too small, the latter a consequence of too little DNA. This set of experiments identifies the antibody-transposome conjugates with optimal activity for chromatin substrates and which chemistry is optimal for the generation of additional antibody-transposase conjugates, such as a non-immune IgG-transposase negative control required for the TAM-ChIP protocol described below.

The optimal conjugate for each of the two antibodies (RNA polyermase II and H3K4tm) is used in the following subsequent experiments **(****Figure 8****, columns 2 through 4)** designed to optimize the effects of different antibody-transposase concentrations, antibody-chromatin incubation times, and sample dilution on transposition efficiency and fragment size. The conditions yielding optimal results are carried forward in the subsequent rounds of procedure optimization. Antibody-transposase concentrations are varied in a two fold dilution series consisting of 2X, 1X and 0.5X; incubations of chromatin with antibody-transposase conjugates are varied for 0.5, 1, 2, and 4 hours; and sample dilution prior to transposase activation to ensure intra-complex transposition are varied as five-fold dilution series (1:X; 1:5X; 1:25X, and 1:125X, where X represents the minimal dilution factor established in Aim 1). The ranges of variables are expanded as warranted based on observed fragment size and transposition efficiency. These experiments identify the conditions which produce a minimum of 500 ng of <200bp tagged-DNA fragments following 18cycles of PCR-amounts required for the Illumina sequencing platform. These experiments result in an optimized TAM-ChIP methodology.

### Example 4: Validation of NGS libraries generated by TAM-ChIP

The DNA libraries produced by the optimized method in developed in the preceding experiments with IgG, RNA polymerase II, and H3K4tm antibody-transposome conjugates are compared with the libraries produced via traditional ChIP-Seq performed with the same unconjugated antibodies. For traditional ChIP-Seq, HeLa chromatin extracts generated for the above set of experiments are incubated with 5ug antibody for 16 hours at 4°C. 1 ug are left unprocessed and serve as the input control. Antibody-chromatin complexes are captured using protein A coated magnetic beads, washed, eluted, and DNA purified following established procedures. ChIP with 5ug of non-immune rabbit IgG is performed in parallel as an antibody specificity control. The ChIP-enriched and the untreated sonicated gDNA are processed according standard protocols for library preparation for sequencing in the Illumina Genome Analyzer GAIl. This consists of end-repair, adaptor ligation, size-selection and PCR amplification, and all these steps are done and sequencing performed according to standard methods. The generated data from both TAM-ChIP and traditional ChIP from two independent experiments is analyzed. Reads mapped to the human genome (alignments) are analyzed to find genomic regions with significant enrichments ("peaks") over alignments obtained from either Input or IgG control DNA. Dozens of H3K4tm and RNA Polymerase II ChIP-Seq assays are performed and analyzed, and very similar results are obtained with the peak calling algorithms MACS [32], SICER [33], or CCAT[34]. In addition, software is used to extend the read alignments to the actual length of the DNA fragments (∼200-250 bp), and to generate a "signal map" showing alignment ("tag") densities in 32-bp bins across the genome and reproducibility between replicates is typically ∼80%. Peaks and signal maps are entered into gene annotation and sample comparison software, returning concise Excel tables showing peak metrics and location of peaks relative to genes. These are used to compare the representation of genomic sequences in the DNA libraries prepared by two methods and show concordance of genomic coverage.

### Example 5: Additional TAM ChIP Embodiments

The methods established above will be recognized by those of ordinary skill in the art to be readily carried out in other embodiments, e.g., (a) those comprising antibodies from different animal hosts (rabbit, mouse, rat and goat) specific for proteins associated with either transcriptionally active euchromatin or transcriptionally silenced heterochromatin (i.e. HP1 proteins, and heterochromatin-associated histone marks), (b) TAM-ChIPs wherein antibody-transposase conjugates are be used singly or simultaneously, and with different degrees of complexity (two-plex, three-plex, etc.), including versions with each conjugate bearing a unique bar-code sequence for antibody identification, (c) those where the antibody-oligonucleotide conjugates prepared above are used in a multiple proximity ligation method (see, e.g., Example 6, below). Antibody-oligo conjugates bound to chromatin are diluted, followed by proximity ligation of the antibody-associated oligonucleotide with the associated chromatin fragment end and nicks sealed. Ligation of oligonucleotides to chromatin has been used to map chromatin higher order structures [35], where co-associating chromatin ends in isolated complexes containing higher-order structures are tagged via ligation with primers and then ligated to each other via their proximity, supporting the feasibility of this approach. Use of a reversible antibody-oligonucleotide cross-linking chemistry or the inclusion of a rare restriction endonuclease cleavage site allows libration of the antibody from the DNA now tagged with the bar-code containing oligonucleotide which is then directly amplified for NGS using an appropriate PCR amplification strategy.

### Example 6: Antibody-Oligonucleotide Conjugates and Proximity Ligation.

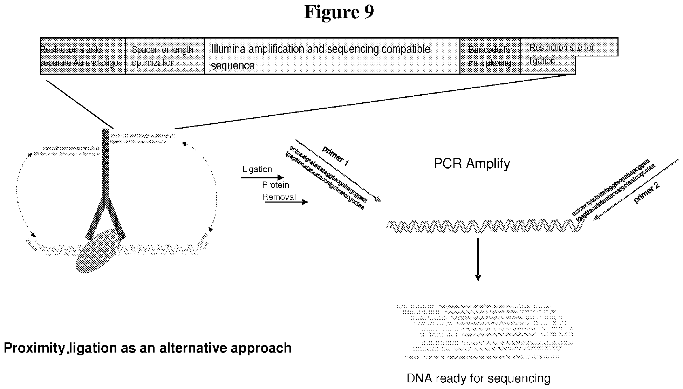

These methods use cross-linked and sonicated (or restriction digested) chromatin as a starting material. Instead of conjugation to transposase, this approach uses conjugation of an antibody to short double-stranded DNA oligonucleotides of known sequence. The conjugate is incubated with cross-linked chromatin that has been either restriction enzyme digested or sonicated, resulting in antibody binding at the intended target. Proximity-mediated ligation is performed, resulting in ligation of the antibody delivered oligos to the target-associated free genomic DNA ends (Figure 9). Digestion of the cross-linked chromatin and the proximity ligation strategy using sonicated or restriction enzyme digested cross-linked chromatin is well established, and these methods build upon and inventively improve on those used in the 5C and Hi-C technologies (Dostie and Dekker, 2007; Lieberman-Aiden et *al*., 2009). The key to the ligation is to perform this step under diluted conditions which favor the interaction of DNA molecules held in close proximity. Following ligation, cross-links can be reversed by heating to 65°C overnight, and proteins can be removed with proteinase K treatment. The regions of interest (e.g., the regions bound by the protein of interest and targeted by the antibody) can be enriched by PCR using primers that anneal to regions within the ligated oligonucleotide sequence, and the resulting amplified DNA can be sequenced using, for example, the Illumina platform, resulting in a genome wide protein binding profile. In addition, this approach is well suited to generate binding profiles of multiple factors in the same sample. This is achieved by designing multiple oligonucleotides, each containing a unique bar code sequence, and conjugating these unique oligos to different antibodies. Multiple antibody conjugates can be added to the same sample at the same time. After sequencing, the data for the multiple targets can be sorted based on the bar code sequence.
Oligonucleotide embodiments. Several features can be designed into the oligonucleotide(s) that are conjugated to the antibody(ies). These features are listed below and depicted in Figure 9.
1. The oligonucleotide is double-stranded and the 5' end of one of the strands is linked to biotin (or a member of different high affinity binding pair). The biotin is used for conjugation to the antibody.
2. There is a restriction site (e.g., Not 1, a "freeing" restriction enzyme in the context of the invention) encoded in each oligonucleotide to allow the oligonucleotide to be separated from the antibody, if needed.
3. There is a region of sequence included that functions only for the purpose of varying the oligonucleotide length. The ligation of the oligonucleotide to the free genomic ends of the captured DNA may be dependent on the length of the oligonucleotides. The entire oligonucleotide is typically about 80 nucleotides in length, although longer or shorter lengths may be optimal in a given application.
4. A region is included that is complementary to Illumina (or other suitable) primers. This region facilitates amplification of oligonucleotide-ligated genomic DNA, preferably to be compatible with sequencing on the intended (e.g., Illumina) platform.
5. There is a 4 base pair (or shorter or longer) barcode. Several different oligonucleotides can be synthesized, each having a different bar code. Oligos with different bar codes can be conjugated to different antibodies, thus allowing multiple antibodies to be used in the same reaction.
6. There is a restriction-site-compatible overhang that allows the oligonucleotide to be ligated to restriction-digested genomic DNA. The overhang may preferably be a 4 nucleotide overhang (e.g., GATC, which is compatible with Dpn II,Mbo I, and Sau3A I, digestions). In such cases, the genomic DNA is cut with a restriction enzyme that having a 4 bp recognition site, which should on average cleave the DNA every 256 bases. Alternatively, a combination of restriction enzymes having 6 bp recognition sites can be used. Alternatively, TA cloning can be used. In such embodiments, sonicated DNA is used which has gone through end repair and A overhang addition. The oligonucleotides are designed to have T overhangs.

### Example 7: Alternate Antibody/Oligonucleotide Conjugation Embodiments

Any suitable chemistry can be used to achieve the antibody/oligonucleotide conjugations used in this invention. One such approach is described below.
1) The biotinylated forward strand oligonucleotide is annealed to the unbiotinylated reverse strand using standard procedures.
2) The antibody can be biotinylated using a number of available kits, for example, the Solulink Chromalink One-Shot biotinylation kit, which allows for quantitation of the number of biotins per antibody and thus allows for optimization of the number of biotins conjugated to the antibody.
3) Self assembly of the conjugate can be achieved by mixing appropriate ratios of the biotinylated oligo, biotinylated antibody, and free streptavidin, a tetramer with four biotin binding sites all of which can be simultaneously occupied.
4) Uncongugated antibody and oligo can be removed using streptavidin magnetic beads.

This approach has been used and validated using a ratio of 2:1:2 (oligo: free streptavidin:antibody). An anti-Goat IgG antibody was coupled to a 100 bp oligo by mixing in the presence of free streptavidin. A goat antibody serves as the antigen and was absorbed to maxisorp 96 well plates at different concentrations. The antibody/oligionucleotide conjugate was allowed to bind the antigen and excess antibody was washed away. After washing, signal was detected using PCR with primers that anneal within the conjugated oligonucleotide.

Those of skill in the art will recognize that many equivalent antibody/oligonucleotide conjugation strategies could be substituted for use in the invention. For example, direct via a chemical cross-linker, indirect via other proteins/biomolecules that have strong interactions, including a streptavidin-protein A fusion protein (or protein G). Protein A binds the antibody in a manner that is known not to interfere with antibody function. A single protein A/G immunoglobulin binding domain could be also used, and expressed as a fusion protein. This would then bind with biotinylated oligonucleotides. There are also biotin-binding peptides that are much smaller than the streptavidin protein.

### REFERENCES CITED

1. Allis, et al., Overvieuv and Concepts, in Epigenetics, Allis, et al., Eds. 2006, Cold Spring Harbor Laboratory Press: New York. p. 23-62.
2. Luger, et al., Nature, 1997. 389(6648): p. 251-60.
3. Strahl and Allis, Nature, 2000. 403(6765): p. 41-5.
4. Grewal and D. Moazed, Science, 2003. 301(5634): p. 798-802.
5. Jenuwein and Allis, Science, 2001. 293(5532): p. 1074-80.
6. Suganuma and Workman, Cell, 2008.135(4): p. 604-7.
7. Grewal, S.I., Current Opinions in Genetics & Development, 2010. 20(2): p. 134-41.
8. Vire, et al., Nature, 2006. 439(7078): p. 871-4.
9. Jones, et al., Nature Genetics, 1998. 19(2): p. 187-91.
10**.** Felsenfeld, G., A Brief History of Epigenetics, in Epigenetics, Allis, et al., Eds. 2006, above, p. 15-22.
11. Braunstein, et al., Genes & Development, 1993. 7(4): p. 592-604.
12. Alberts, et al., Cell, 1998. 92(4): p. 475-87.
13. Rister and Desplan, Bioessays, 2010. 32(5): p. 381-4.
14. Active Motif, I., ChIP-IT Express Magnetic Chromatin Immunoprecipitation Kit. 2011, Active Motif, Carlsbad, California, USA.
15. Mizuucki and Baker, Chemical Mechansims for Mobilizing DNA, in Mobile DNA II, Craig, et al., Eds. 2002, ASM Press: Washington, p. 12-23.
16. Goryshin and Reznikoff, Journal of Biological Chemistry, 1998. 273(13): p. 7367-74.
17. Davies, et al., Science, 2000. 289(5476): p. 77-85.
18. Goryshin, et al., PNAS USA, 1998. 95(18): p. 10716-21.
19. Goryshin, et al., Nature Biotechnology, 2000. 18(1): p. 97-100.
20. Gallagher, et al., PNAS USA, 2007. 104(3): p. 1009-14.
21. Vidal, et al., PLoS One, 2009. 4(7): p. e6232.
22. Bertram, et al., Nucleic Acids Research, 2005. 33(18): p. e153.
23. Shi, et al., Moleucular and Biochemical Parasitology, 2002. 121(1): p. 141-4.
24. Suganuma, et al., Biology of Reproduction, 2005. 73(6): p. 1157-63.
25. Steger, et al., Molecular and Cellular Biology, 2008. 28(8): p. 2825-39.
26. Dirksen and Dawson, Bioconjugate Chemistry, 2008. 19(12): p. 2543-8.
27. Fredriksson, et al., Clinical Chemistry, 2008. 54(3): p. 582-9.
28. Jarvius, et al., Molecular and Cellular Proteomics, 2007. 6(9): p. 1500-9.
29. Solulink, Protein-Protein Conjugation Kit Solulink Inc.: San Diego.
30. Mahnke Braam and Reznikoff, Journal of Biological Chemistry, 1998. 273(18): p. 10908-13.
31. Mahnke Braam, et al., Journal of Biological Chemistry, 1999. 274(1): p. 86-92.
32. Zhang, et al., Genome Biol, 2008. 9(9): p. R137.
33. Zang, et al., Bioinformatics, 2009. 25(15): p. 1952-8.
34. Xu, et al., Bioinformatics, 2010. 26(9): p. 1199-204.
35. Li, et al., Genome Biology, 2010. 11(2): p. R22.
36. Life Science Tools and Reagents: Global Markets 2011. 2011, BCC Research, Inc., Wellesley, MAm USA.
37. Epigenetics Market Trends 2011. 2011, Select BioSciences, Ltd, Sudbury, UK. 38. Lieberman-Aiden et al., Science, 2009. 326 (5950):p. 289-293.

## Claims

1. An antibody-transposome complex, comprising an antibody that binds a target nucleic acid-associated protein or protein domain chemically crosslinked to a transposome that comprises a transposase and a transposon cassette.

2. A method for tagging chromatin with an antibody-transposome complex according to claim 1 comprising
a. extracting and fragmenting chromatin from a prepared sample;
b. adding at least one antibody-transposome complex, wherein the antibody binds a target nucleic acid-associated protein;
c. allowing said antibody(ies) to locate at its/their target protein(s) in said chromatin fragments;
d. activating the transposase resulting in insertion of the transposon cassette proximally into the antibody-target nucleic acid-associated protein associated DNA fragment to tag the DNA fragments.

3. The method according to claim 2, wherein the chromatin is cross-linked.

4. The method according to claim 3 further comprising step
e. removing the cross-links.

5. The method according to claim 4, further comprising step
f. enriching regions bound by the protein targeted by the antibody using polymerase chain reaction (PCR).

## Patentansprüche

1. Antikörper-Transposomkomplex, einen Antikörper umfassend, der ein Ziel-Nukleinsäure-dazugehöriges Protein oder eine Proteindomäne bindet, die chemisch mit einem Transposom vernetzt ist, wobei das Transposom eine Transposase und eine Transposonkassette umfasst.

2. Verfahren zum Markieren von Chromatin mit einem Antikörper-Transposomkomplex gemäß Anspruch 1, umfassend
a. Extrahieren und Fragmentieren von Chromatin aus einer vorbereiteten Probe;
b. Hinzufügen von mindestens einem Antikörper-Transposomkomplex, wobei der Antikörper ein Ziel-Nukleinsäure-dazugehöriges Protein bindet;
c. Erlauben für besagte(n) Antikörper, sich an seinem/ihren Zielprotein(en) in besagten Chromatin-Fragmenten zu fixieren;
d. Aktivieren der Transposase, was darin resultiert, dass die Transposonkassette proximal in das zum Antikörper-Ziel-Nukleinsäure-dazugehörigen Protein dazugehörige DNA-Fragment eingefügt wird, um die DNA-Fragmente zu markieren.

3. Das Verfahren gemäß Anspruch 2, wobei das Chromatin vernetzt wird.

4. Das Verfahren gemäß Anspruch 3, des Weiteren umfassend den Schritt
e. Entfernen der Vernetzungen.

5. Das Verfahren gemäß Anspruch 4, des Weiteren umfassend den Schritt
f. Anreichern von Regionen, die durch das Protein gebunden werden auf das der Antikörper abzielt, unter Verwendung von Polymerase-Kettenreaktion (PCR).

## Revendications

1. Un complexe anticorps-transposome, comprenant un anticorps qui se lie à une protéine associée à un acide nucléique cible ou un domaine protéique chimiquement réticulé à un transposome qui comprend une transposase et une cassette transposon.

2. Un procédé de marquage de la chromatine avec un complexe anticorps-transposome selon la revendication 1, consistant à
a. extraire et fragmenter la chromatine à partir d'un échantillon préparé ;
b. ajouter au moins un complexe anticorps-transposome, dans lequel l'anticorps se lie à une protéine associée à un acide nucléique cible ;
c. permettre audit ou auxdits anticorps de se localiser au niveau de sa ou leur protéines cibles dans lesdits fragments de chromatine ;
d. activer la transposase entraînant l'insertion de la cassette transposon de manière proximale dans le fragment d'ADN associé à une protéine associée à un acide nucléique cible d'anticorps pour marquer les fragments d'ADN.

3. Le procédé selon la revendication 2, dans lequel la chromatine est réticulée.

4. Le procédé selon la revendication 3, comprend en outre l'étape
e. retirer les réticulations.

5. Le procédé selon la revendication 4, comprenant en outre l'étape
f. enrichir les régions liées par la protéine ciblée par l'anticorps en utilisant une réaction en chaîne par polymérase (PCR)
